# EUROPEAN PATENT APPLICATION

(11) **EP 3 714 912 A1**
(43) Date of publication of application: **30.09.2020**
(21) Application number: 20166283.0
(22) Date of filing: 27.03.2020
(51) Int. Cl.: A61L 27/44, A61L 27/46, A61L 27/48, A61L 27/58

(54) **GEL GRAFT MATERIAL**

(30) Priority: 27.03.2019 LB 1164319
(71) Applicant: Khalil, Wael Hassan, Beirut (LB)
(72) Inventor: Khalil, Wael Hassan, Beirut (LB)
(74) Representative: Viering, Jentschura & Partner mbB Patent- und Rechtsanwälte

(57) **Abstract**

A gel graft material (GGM) comprising a biopolymer matrix component, a biopolymer filler component, and a bioactive mineral component.

## Description

### FIELD OF THE INVENTION

The technical field generally relates to gel-like or paste-like bone graft materials. In particular, the present disclosure is concerned with a bone regeneration, such as a bone augmentation or bone grafting.

### BACKGROUND OF THE INVENTION

Bone is dynamic, highly vascularized tissue with a unique capacity to heal and remodel. This living tissue is a highly specialized hard form of connective tissue that forms most of the skeleton and is the chief tissue of the body. Its main role is to provide structural support for the body. Furthermore, the skeleton also serves as a mineral reservoir, supports muscular contraction resulting in motion, withstands load bearing and protects internal 15 organs. Bone as dynamic living tissue shows marked structural alteration in response to loading changes of stress and to vascular, endocrine, and nutritional influences. Bone tissue in the adult skeleton is arranged in two architectural forms: trabecular, also called cancellous or spongy bone, and cortical or compact bone. The proportions of these two 20 architectural forms differ at various locations in the skeleton. Cortical bone is almost solid, being only S10% porous. Trabecular bone presents a higher porosity, 50%-90%, making its modulus and ultimate compressive strength around 20 times lower than that of cortical bone.

Synthetic bone has been used for bone reconstruction. The use of synthetic substitutes. Various materials have been investigated for constructing bone; including, without limitation, ceramics, polymers and composites of biomaterials. Whilst current synthetic bone materials have achieved considerable success in terms of use in various medical procedures, there remains an ongoing need for improved bone grafting materials that are biocompatible, promote healing, able to be manufactured efficiently and shaped as desired and easy to handle and manipulate. Thus, it is desired to provide a bone graft material, specifically synthetic bone composite that allows at least some of the aforementioned objectives to be achieved.

Hence, there is a need to provide improved bone grafting materials.

### SUMMARY OF THE INVENTION

This need is met by the products, methods, uses and further embodiments of the present invention in providing for a gel-like or paste-like bone graft material that may be hardened to yield a hardened bone graft material.

In a first aspect, the present invention thus relates to a bone graft material, comprising a biopolymer matrix component, a biopolymer filler component, and a bioactive mineral filler component.

In a second aspect, the present invention is directed to a method for manufacturing a bone graft material as herein described, comprising the steps of:
i) providing a mixture comprising a biopolymer matrix component and a biopolymer filler component in powder form, and a bioactive mineral filler component in particulate form;
ii) mixing the said components with water to obtain a gel-like or paste-like material; and, optionally,
iii) hardening the mixture obtained after step ii) to obtain a hardened bone graft material.

In a third aspect, the present invention also relates to a bone graft material as herein described or a bone graft material obtainable in a method described herein for use in bone grafting related to dental implant placement or dental implant surgery, wherein, preferably, the dental implant surgery comprises craniofacial and/or maxillofacial bone surgery, and the dental implant surgery comprises sinus grafting, ridge augmentation and/or preservation.

Finally, the present invention is also directed, in a further aspect, to a kit-of-parts comprising a biopolymer matrix component, a biopolymer filler component, and a bioactive mineral filler component, characterized in that the biopolymer matrix component and the biopolymer filler component are provided in powder form and the bioactive mineral filler component is provided in particulate and/or powder form, the kit optionally further comprising water, more preferably deionized water.

These and other aspects of the invention will be apparent from and elucidated with reference to the embodiment(s) described hereinafter as well as the dependent claims.

### BRIEF DESCRIPTION OF THE DRAWINGS

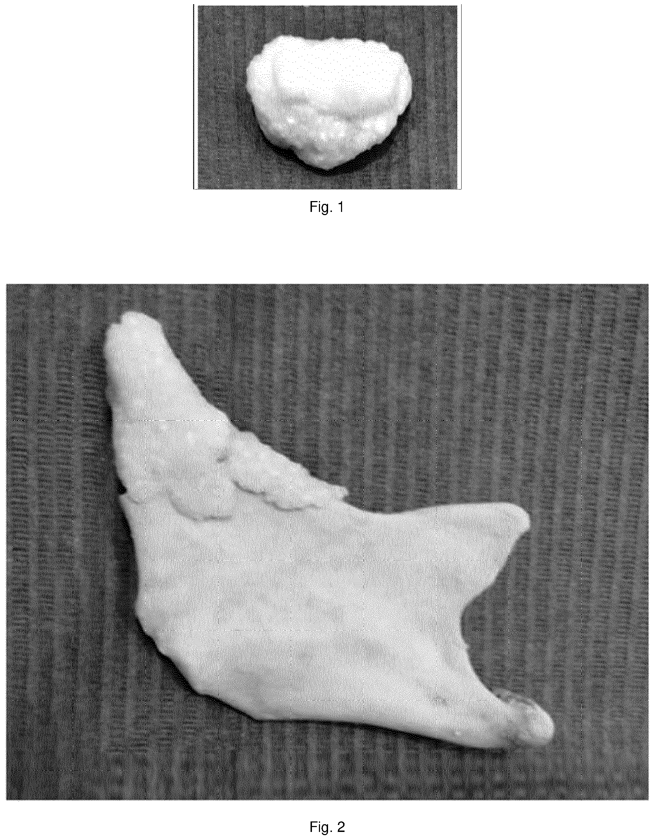

**Figure 1****:** Gel graft material according to the present invention. The material consists of a mixture of equal amounts of biopolymer matrix component (CMC), biopolymer filler component PLA and bioactive mineral filler component (consisting of CaSO₄ and βTCP); and water, wherein the ratio of water and said mixture is 1:2.
**Figure 2****:** The gel graft material according to the present invention used to reconstruct bone defect (dry jaw model). The graft material consists of a mixture of equal amounts of biopolymer matrix component (CMC), biopolymer filler component (PLA) and bioactive mineral filler component (consisting of CaSO₄ and βTCP); and water, wherein the ratio of water and said mixture is 1:2.

### DETAILED DESCRIPTION OF THE INVENTION

The following specific description is merely exemplary in nature and is not intended to limit the application and uses. Furthermore, there is no intention to be bound by any expressed or implied theory presented in the preceding technical field, background, brief summary or the following detailed description.

Unless otherwise defined, all technical and scientific terms used herein have the same meaning as commonly understood by one of ordinary skill in the art.

In the present specification, the terms "a" and "an" and "at least one" are the same as the term "one or more" and can be employed interchangeably.

"One or more", as used herein, relates to at least one and comprises 1, 2, 3, 4, 5, 6, 7, 8, 9 or more of the referenced species. Similarly, "at least one" means one or more, i.e. 1, 2, 3, 4, 5, 6, 7, 8, 9 or more.

"At least one", as used herein in relation to any component, refers to the number of different entities, such as chemically different molecules, i.e. to the number of different types of the referenced species, but not to the total number of entities (such as molecules).

Numeric values specified without decimal places here refer to the full value specified with one decimal place, i.e., for example, 99 % means 99.0 %, unless otherwise defined.

"About" and "approximately", as used herein in relation to a numerical value, refer to said value ±10%, preferably ±5%.

All percentages given herein in relation to the compositions or formulations relate to weight % relative to the total weight of the respective composition or formula, if not explicitly stated otherwise.

According to the present invention, the bone graft material, comprising a biopolymer matrix component, a biopolymer filler component, and a bioactive mineral filler component.

In various embodiments, the biopolymer matrix component comprises or consists of at least one polysaccharide. Non-limiting examples of polysaccharides suitable for employment in the context of the present invention include natural polysaccharides and derivatives thereof, particularly amylose, maltodextrin, amylopectin, starch, dextran, hyaluronic acid, heparin, chondroitin sulfate, dermatan sulfate, heparan sulfate, keratan sulfate, dextran sulfate, pentosan polysulfate, chitosan, and carboxymethyl cellulose (CMC). In various embodiments, the biopolymer matrix component is prepared from cellulose. According to preferred embodiments, the biopolymer matrix component comprises or consists of carboxymethyl cellulose (CMC).

In various embodiments, the biopolymer filler component comprises or consists of poly lactic acid (PLA), poly-ε-caprolactone, polyglycolic acid, or copolymers of two or more of the aforementioned. According to preferred embodiments, the biopolymer filler component comprises or consists of poly lactic acid (PLA). According to particularly preferred embodiments, the biopolymer filler component consists of poly lactic acid (PLA).

In various embodiments, the bioactive mineral filler component comprises or consists of calcium sulphate (CaSO₄) and/or calcium sulphate hydrates and/or calcium phosphate compounds and/or salts thereof, such as, without limitation, calcium phosphate ceramics, such as for instance, but without limitation, tricalcium phosphates, such as alpha-tricalcium phosphate (αTCP) and beta-tricalcium phosphate (βTCP). According to preferred embodiments, the bioactive mineral filler component comprises or consists of calcium sulphate (CaSO₄) and/or beta-tricalcium phosphate (βTCP), preferably both. In various embodiments, the amount of calcium sulphate (CaSO₄) and/or calcium sulphate hydrates may be in the range of about 10 to about 90 vol.-%, preferably in the range of about 20 to about 80 vol.-%, more preferably in the range of about 30 to about 70 vol.-%, even more preferably in the range of about 40 to about 60 vol.-%, such as bout 40, 45, 50, 55 or 60 vol.-%, based on the total volume of the bioactive mineral filler component. In various embodiments, the amount of calcium phosphate compounds and/or salts thereof, preferably beta-tricalcium phosphate (βTCP), may be in the range of about 10 to about 90 vol.-%, preferably in the range of about 20 to about 80 vol.-%, more preferably in the range of about 30 to about 70 vol.-%, even more preferably in the range of about 35 to about 60 vol.-%, such as bout 35, 40, 45, 50, 55 or 60 vol.-%, based on the total volume of the bioactive mineral filler component, even more preferably in the range of about 35 to about 45 vol.-%, such as bout 35, 36, 37 ,38, 39, 40, 41, 42, 43, 44 or 45 vol.-%, based on the total volume of the bioactive mineral filler component. In particularly preferred embodiments, the bioactive mineral filler component consists of calcium sulphate (CaSO₄) and beta-tricalcium phosphate (βTCP). According to various embodiments, the ratio of calcium sulphate (CaSO₄) to beta tricalcium phosphate (βTCP) is about 20:80 or about 60:40, preferably about 60:40. In various other embodiments, the ratio of carboxymethyl cellulose (CMC) to of calcium sulphate (CaSO₄) is about 40:60, more preferably about35:65.

In various embodiments, the bone graft material according to the present invention comprises the biopolymer matrix component in an amount of about 15 to about 40 vol.-%, preferably in an amount of about 20 to about 30 vol.-%, such as about 20, 21, 22, 23, 24, 25 or 30 wt.-%, based on the total weight of the bone graft material.

In various embodiments, the bone graft material according to the present invention comprises the biopolymer filler component in an amount of about 1 to about 20 vol.-%, preferably in an amount of about 1 to about 11 vol.-%, such as about 1, 2, 3, 4, 5, 6, 7, 8, 9, 10 or 11 wt.-%, based on the total weight of the bone graft material.

In various embodiments, the bone graft material according to the present invention comprises the bioactive mineral filler CaSO₄ component in an amount of about 20 to about 65 vol.-%, preferably in an amount of about 30 to about 40 vol.-%, such as about 30, 31, 32, 33, 34, 35, 36, 37, 38, 39 or 40 wt.-%, based on the total weight of the bone graft material.

In various embodiments, the bone graft material according to the present invention comprises the bioactive mineral filler βTCP component in an amount of about 20 to about 40 vol.-%, preferably in an amount of 25 to 35 vol.-%, such as about 25, 26, 27, 28, 29, 30, 31, 32, 33, 34 or 35 wt.-%, based on the total weight of the bone graft material.

In exemplary embodiments, the biopolymer matrix component, preferably CMC; the biopolymer filler component, preferably PLA; and the bioactive mineral filler component, preferably consisting of CaSO₄ and βTCP, are each independently present in the bone graft material in an amount of at least about 1 vol.-%, preferably at least about 5 vol.-%, such as about 5, 6, 7, 8, 9, 10, 11, 12, 13, 14, 15, 16, 17, 18, 19 or 20 vol.-%, preferably at least about 10 vol.-%, such as about 10, 11, 12, 13, 14, 15, 16, 17, 18, 19, 20, 21, 22, 23, 24, 25 vol.-%, based on the total volume of the bone graft material.

According to various embodiments, the biopolymer matrix component, preferably CMC; the biopolymer filler component, preferably CaSO₄; and the bioactive mineral filler component, preferably consisting of PLA and βTCP, are present within the bone graft material herein described in equal amounts.

In various embodiments, the bone graft material of the present invention further comprises water, preferably distilled or deionized water or a mixture thereof. In various embodiments, the content of water in the herein described bone graft material is in the range of about 1% vol.-%, preferably at least about 5 vol.-%, preferably at least about 10 vol.-%, based on the total volume of the bone graft material.

According to various embodiments, the ratio of water to solid components as described above, i.e. the biopolymer matrix component, the biopolymer filler component, and the bioactive mineral filler component, is about 1:5 to about 3:1, preferably about 1:3 to about 2:1, more preferably about 1:2 to about 1:1, such as about 1:2 or about 1.5:2, particularly if the ratio of biopolymer matrix component, biopolymer filler component, and bioactive mineral filler component is about 1:1:1.

In comprising the at least one biopolymer matrix component, as herein defined, the at least one biopolymer filler component, as herein defined, the at least one bioactive mineral filler component, as herein defined, and water, each in the herein-specified amounts and ratios, the bone graft material according to the present invention has a gel-like or paste-like consistency, which allows for easy handling, forming, manipulating and insertion into the respective recipient site. Thus, according to various embodiments, the bone graft material of the present invention is a gel-like or paste-like bone graft material, i.e. it has a gel-like or paste-like consistency at about 20 °C and about 1.013 bar.

In various embodiments, the bone graft material of the present invention further comprises at least one additional component, such as to enhance bone regeneration, preferably selected from but not limited to the group consisting of osteogenic, osteoconductive and/or osteoinductive materials, non-limiting examples of which include, without limitation, collagen, hyaluronic acid and derivatives of the aforementioned, as well as (autogenous) bone material, such as (autogenous) bone chips, powders and particulates; anti-inflammatory agents; antibiotics; anti-viral agents; anti-cancer agents; immunosuppressants; enzymes inhibitors; hormones; and proteins, such as bone morphogenic proteins.

Amounts of the aforementioned, non-limiting examples of further components suitable for employment according to the present invention are not particularly limited and may in the range of about 0.0001 to about 10 wt.-%, such as about 0.001 to about 5 wt.-%, based on the total weight of the bone graft material herein described.

The term "osteogenic", as used herein, refers to the ability of an agent, material, or implant to enhance or accelerate the growth of new bone tissue by one or more mechanisms such as osteogenesis, osteoconduction, and/or osteoinduction.

The term "osteoconductive", as used herein, refers to the ability of a non-osteoinductive substance to serve as a suitable template or substance along which bone may grow.

The term "osteoinductive", as used herein, refers to the quality of being able to recruit cells from the host that have the potential to stimulate new bone formation. Any material that can induce the formation of ectopic bone in the soft tissue of an animal is considered osteoinductive. For example, most osteoinductive materials induce bone formation in athymic rats when assayed according to the method of Edwards et al., "Osteoinduction of Human Demineralized Bone: Characterization in a Rat Model," Clinical Orthopaedics & Rel. Res., 357:219-228, December 1998, incorporated herein by reference.

According to various embodiments, the bone-graft material according to the present invention comprises only biocompatible materials and components, i.e. is essentially free of non-biocompatible components.

Biocompatible, as used herein, refers to materials that, upon administration *in vivo,* do not induce undesirable long-term effects.

According to various embodiments, the bone-graft material according to the present invention comprises only biodegradable materials and components, i.e. is essentially free of non-biodegradable components.

According to various embodiments, the biopolymer matrix is biodegradable within a period of about 60 weeks.

In various embodiments, the biopolymer filler is biodegradable in more than 30 weeks, such as, for instance, in more than 31, 32, 33, 34, 35, 36, 37, 38, 39, 40, 45 or 50 weeks.

In various embodiments, the bioactive mineral filler, particularly the bioactive mineral filler material beta tricalcium phosphate, is biodegradable within a period of about 16 to about 24 weeks, such as, for instance, within a period of about 18 to about 22 weeks. According to various embodiments, the bioactive mineral filler material CaSO₄ is biodegradable in less than about 8 weeks.

In various embodiments, the bone graft material of the present invention may further include at least one crosslinking agent and optionally at least one crosslinking catalyst, preferably selected from hyaluronic acid and carbodiimide, or citric acid. According to various embodiments, suitable crosslinking systems allow for hardening/curing of the herein described gel-like or paste-like bone graft material upon exposure to electromagnetic radiation, such as UV radiation, or *via* chemical crosslinking effected by hyaluronic acid and carbodiimide, or by citric acid.

Suitable agents and methods for crosslinking of gel-like or paste-like materials, as herein disclosed, are generally known in the art. For instance, but without limitation, crosslinking of cellulose derivatives and hyaluronic acid with water-soluble carbodiimide is generally disclosed and described in Demitri, C.; Del Sole, R.; Scalera, F.; Sannino, A.; Vasapollo, G.; Maffezzoli, A.; Ambrosio, L.; Nicolais, L, Novel superabsorbent cellulose-based hydrogels crosslinked with citric acid. J. Appl. Polym. Sci. 2008, 110 (4), 2453-2460.

Methods and agents for the crosslinking of cellulose-based hydrogels with citric acid are disclosed and discsribed in Sannino, A.; Pappadà, S.; Madaghiele, M.; Maffezzoli, A.; Ambrosio, L.; Nicolais, L. Crosslinking of cellulose derivatives and hyaluronic acid with water-soluble carbodiimide, Polymer 2005, 46 (25), 11206-11212. The content of both documents shall be understood as being incorporated herein by reference.

Crosslinking may be promoted and assisted *via* exposure to, for instance, ultrasound or electromagnetic radiation having a wavelength of about 1mm to about 1 m and/or a frequency of about 300 GHz to about 300 MHz, for instance for about 10 to 30 seconds.

Hardening of the gel-like or paste-like bone graft material herein described yields a more rigid and resilient, less-compressible bone graft material(hydrogel). Advantageously, the hardening of the gel-like bone graft material herein described may be effected after moulding, forming, fitting and optionally implanting of the bone graft and can be applied in various clinical applications such as huge bone defect in oral, craniofacial or orthopaedic surgeries when a harder consistency of the material is needed.

In a further aspect, the present invention further provides for a method of manufacture of a bone graft material as herein described, said method comprising the steps of:
i) providing a mixture comprising a biopolymer matrix component, as herein defined, and a biopolymer filler component, as herein defined, preferably in powder form, and a bioactive mineral filler component, as herein defined, preferably in particulate and/or form, wherein preferably said particles have a mean diameter in the range of between about 1 µm to about 10 µm; and
ii) mixing said components with water to obtain a gel-like or paste-like material.

Naturally, mixing, in the context of the present invention, may include and be promoted by agitation of the respective components, for instance using a spatula or a magnetic stirrer and the like.

In various embodiments, the biopolymer matrix component comprises or consists of carboxymethyl cellulose (CMC). In various embodiments, the biopolymer filler component comprises or consists of poly lactic acid (PLA). In various embodiments, the bioactive mineral filler component comprises or consists of calcium sulphate (CaSO₄) and/or beta tricalcium phosphate (βTCP), preferably both. According to preferred embodiments, the ratio of calcium sulphate (CaSO₄) to beta tricalcium phosphate (βTCP) is about 20:80 or about 60:40, preferably about 60:40. In various embodiments, the ratio of carboxymethyl cellulose (CMC) to of calcium sulphate (CaSO₄) is about 40:60, preferably about 35:65.

According to various embodiments, CaSO₄ is provided in powder form and beta tricalcium phosphate is provided in particulate form.

In various embodiments, the biopolymer matrix component, the biopolymer filler component and the bioactive mineral filler component are, independently of each other, present in the mixture obtained in step i) in an amount of about 1 to about 65% vol.-%, preferably in an amount of about 5 to about 35 vol.-%, more preferably in an amount of about 10 to about 33 vol.-%, based on the total volume of said mixture. According to preferred embodiments, said components are present in the mixture obtained in step i) in equal amounts.

According to various embodiments, the ratio of water to the mixture of biopolymer matrix component, biopolymer filler component, and bioactive mineral filler component, as herein described and defined, is about 1:5 to about 3:1, preferably about 1:3 to about 2:1, more preferably about 1:2 to about 1:1, such as about 1:2 or about 1.5:2, particularly if the ratio of biopolymer matrix component, biopolymer filler component, and bioactive mineral filler component is about 1:1:1. However, the amount of water used may be varied and adapted, such as if further components and ingredients are added, as herein described, to obtain a gel-like bone graft material having the desired viscosity and consistency, which may dependent on, vary and thus be chosen with respect to the intended end-use, i.e. clinical application.

Preferably, the mixing of step ii) comprises exposing the respective mixture, including water, to elevated temperatures, preferably temperatures in the range of about 20 to about 90 °C, preferably about 25 to about 85 °C, more preferably about 30 to about 80 °C, more preferably about 35 to about 75 °C, even more preferably about 40 to about 70 °C, still more preferably about 45 to about 70 °C, most preferably about 50 to about 70 °C, such as about 55 °C to about 70 °C, for instance about 65 °C, such as about 60 °C to about 70 °C, preferably for about 15 s to about 5 h, more preferably for about 30 s to about 2 h, more preferably for about 60 s to about 1 h, more preferably for about 1.5 min to about 30 min.

According to various embodiments, this may include the water having a temperature of about 20 to about 90 °C, preferably about 25 to about 85 °C, more preferably about 30 to about 80 °C, more preferably about 35 to about 75 °C, even more preferably about 40 to about 70 °C, still more preferably about 45 to about 70 °C, most preferably about 50 to about 70 °C, such as about 55 °C to about 70 °C, for instance about 65 °C.

According to various embodiments, the water is distilled or deionized water or a mixture thereof.

In various embodiments, the method further comprises the addition of at least one additional component selected from the group consisting of osteogenic, osteoconductive and/or osteoinductive materials; anti-inflammatory agents; antibiotics; anti-viral agents; anti-cancer agents; immunosuppressants; enzymes inhibitors; and hormones, as described and defined herein above, to the mixture of step i) and/or the mixture of step ii).

In various embodiments, the method of the present invention may further comprise a step iii) of hardening the mixture obtained after step ii) to obtain a hardened bone graft material. In various embodiments, the hardening of step iii) is effected by exposure of the mixture obtained after step ii) to curing/hardening conditions, wherein the nature of said curing/hardening conditions is chosen dependent upon a respective crosslinking agent and optional crosslinking catalyst, as herein described above, added to the mixture of step i) or step ii). According to preferred embodiments, suitable crosslinking systems allow for hardening/curing of the herein described gel-like bone graft material upon expose to radiation, preferably UV radiation, as herein described and defined above.

The gel graft material can be applied directly on bone defect and manipulated and shaped in order to fit and fill the defect either on later aspect of the bone plate or inside the alveolar socket in ridge preservation and also in sinus grafting.

An exemplary method of preparing the gel-like bone graft material herein described comprises providing a first component of CMC powder, providing a second component of calcium sulphate powder, these two powders are mixed together (the ratio of carboxymethyl cellulose (CMC) to of calcium sulphate (CaSO₄) being, for instance, 40:60, then added to equal volume or unequal of particles including the third and fourth component respectively (i.e. βTCP and PLA, the ration of calcium sulphate (CaSO₄) to beta tricalcium phosphate (βTCP) being, for instance, about 20:80; the ratio of CMC to CaSO₄ being, for instance, about 40:60), then the whole mixture of powders and particles is added to water (about 20 °C to about 70 °C) distilled water, the ratio of water to powder/particles mixture being, for instance, about 1:2 or 1.5/2). Optionally, an antibiotic or other therapeutic molecules could be added providing anti-infectious treatment or prophylactic to bone infection. The gel-like bone graft material may be hardened by using a crosslinking agent, wherein curing may, for instance, be effected upon exposure to radiation, such as UV radiation using appropriate UV curing systems, allowing different clinical applications of the material.

As herein described, osteoconductive or osteoinductive compounds and materials can be added to the powder mixture or particles mixture or even incorporated into the gel in order to enhance bone regeneration, for instance by adding autogenous bone chips. In exemplary applications of replacement and/or bone reconstruction, the gel-like bone graft material could be prepared by the clinician before the procedure or even during by mixing hot distilled water to powder/particle mixture. While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration of the disclosure in any way. Rather, the foregoing detailed description will provide those skilled in the art with a convenient road map for implementing the exemplary embodiment or exemplary embodiments. It should be understood that various changes can be made in the function and arrangement of elements without departing from the scope of the disclosure as set forth in the appended claims and the legal equivalents thereof.

The combination of biopolymer matrix, biopolymer filler and bioactive mineral filler in the bone graft material has been found to be able to facilitate bone grafting by using a gel-like or paste-like grafting material instead of using bone particles or ships and sparing the use of a resorbable or non-resorbable membrane (that are usually used to cover the bone graft particles) especially in oral surgery. The use of a gel-like material as the bone grafting material will facilitate the manipulation of bone grafting and increase the success rate of bone grafting by reducing tremendously the operation time and by giving a better stability and fixation of bone grafting to the recipient site and without the need of use of a membrane to cover the bone graft material, which would otherwise complicate surgery in terms of time and potential complications.

Specifically, the matrix claimed offers a biodegradable and biocompatible features. Further, the gel can be accurately and easily shaped and could fill bone defect in a three-dimensional fit due to its adhesive property that may prevent fibrous tissue interface and improve its biological activity and biocompatibility. Without wishing to be bound by theory, it is believed that the biopolymer matrix will hold the other components in a gel-like or paste-like material and that can degrade by time to create porosity that promotes bone ingrowth. Further, biopolymers filler the Poly lactic Acid (PLA) can be selected having intrinsic tissue characteristics relating to slow degradation to prevent soft tissue invasion in bone graft material and play a role as membrane, the slow degradation of PLA give a better stability of the gel-like or paste-like grafting material. Without wishing to be bound by theory, the bioactive mineral filler can form a strong bond with bone, it is bioactive and osteoinductive. Moreover, using CaSO₄ give a more plasticity to the graft and give an antibacterial effect due to alkaline medium along with the bioactive βTCP both can be replaced by bone rapidly and its degradation stimulating bone growth in 4 to 6 months.

The gel-like or paste-like bone graft material is useful in orthopaedic surgery for repairing bone defect, for treating bone tumors and for repairing bone infection by adding antibiotic or other therapeutic molecules in the mixture. The gel or paste graft material may be useful in maxillofacial and craniofacial applications in non-load bearing region. It can also can be used for ridge preservation after dental extraction (for ridge preservations), for bone regeneration in periodontal defects, for sinus grafting, etc..

Thus, in a further aspect, the present invention also relates to a bone graft material, as herein described, or a bone graft material obtainable in a method as herein described for use in bone grafting related to dental implant placement or dental implant surgery, wherein, preferably, the dental implant surgery comprises craniofacial and/or maxillofacial bone surgery, such as, for instance, reconstructive surgery after tumor or cyst enucleation, or even orthopaedic surgery, and the dental implant surgery comprises sinus grafting, ridge augmentation and/or preservation.

Finally, the present invention also relates to a kit-of-parts comprising a biopolymer matrix component, a biopolymer filler component, and a bioactive mineral filler component, each as herein defined as described, characterized in that the biopolymer matrix component and the biopolymer filler component are provided in powder form and the bioactive mineral filler component is provided in particulate and/or powder form, the kit optionally further comprising water, more preferably distilled or deionized water or a mixture thereof.

In various embodiments, said kit-of-parts further comprises at least one crosslinking agent and optionally at least one crosslinking catalyst, preferably selected from preferably selected from hyaluronic acid and carbodiimide, or citric acid.

All embodiments disclosed herein in relation to the bone graft material apply similarly to the methods, uses and other aspects of the invention and *vice versa.*

Many modifications and other embodiments of the inventions set forth herein will come to mind to one skilled in the art to which these inventions pertain having the benefit of the teachings presented in the foregoing descriptions. Therefore, it is to be understood that the inventions are not to be limited to the described drawings and specific embodiments disclosed and that modifications and other embodiments are intended to be included within the scope of the appended claims. Moreover, although the foregoing descriptions describe some example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

The following examples are given to illustrate the present invention. Because these examples are given for illustrative purposes only, the invention should not be deemed limited thereto.

### EXAMPLES

### Example 1: Preparation of gel-like bone graft material

The gel or paste graft material is prepared by adding warm water (such as 20 °C to 60 °C) to a mixture of powders of equal amount of carboxymethyl cellulose, calcium sulfate and particles of βTCP and PLA. The mixture will be stirred for 2 minutes to form a gel or paste that can be used after cooling down to room temperature (i.e. about 20 °C).

## Claims

1. Bone graft material, comprising a biopolymer matrix component, a biopolymer filler component, and a bioactive mineral filler component.

2. The bone graft material according to claim 1, **characterized in that** the biopolymer matrix component
- comprises or consists of at least one polysaccharide; and/or
- is prepared from cellulose; and/or
- comprises or consists of carboxymethyl cellulose (CMC).

3. The bone graft material according to claim 1 or claim 2, **characterized in that** the biopolymer filler component comprises or consists of poly lactic acid (PLA).

4. The bone graft material according to any one of claims 1 to 3, **characterized in that** the bioactive mineral filler component comprises or consists of calcium sulphate (CaSO₄) and/or beta tricalcium phosphate (βTCP), preferably both.

5. The bone graft material according to any one of claims 1 to 4, **characterized in that** the CMC, CaSO₄, PLA and βTCP are each independently present in an amount of at least about 1% vol.-%, preferably at least about 5 vol.-%, preferably at least about 10 vol.-%, based on the total volume of the bone graft material.

6. The bone graft material according to any one of claims 1 to 5, **characterized in that** it further comprises water, preferably in an amount of about 1% vol.-%, preferably at least about 5 vol.-%, preferably at least about 10 vol.-%, based on the total volume of the bone graft material.

7. The bone graft material according to any one of claims 1 to 6, **characterized in that** it further comprises at least one additional component selected from the group consisting of osteogenic, osteoconductive and/or osteoinductive materials; anti-inflammatory agents; antibiotics; anti-viral agents; anti-cancer agents; immunosuppressants; enzymes inhibitors; hormones; and proteins.

8. The bone graft material according to any one of claims 1 to 7, **characterized in that** it further comprises at least one crosslinking agent and optionally at least one crosslinking catalyst, preferably selected from hyaluronic acid and carbodiimide, or citric acid.

9. The bone graft material according to any one of claims 1 to 8, **characterized in that**
- all components are biodegradable; and/or
- the biopolymer matrix is biodegradable within a period of about 60 weeks; and/or
- the biopolymer filler is biodegradable in more than 30 weeks; and/or
- the bioactive mineral filler material CaSO₄ is biodegradable in less than 8 weeks; and/or
- the bioactive mineral filler, particularly the bioactive mineral filler material beta tricalcium phosphate, is biodegradable in 16 to 24 weeks.

10. Method for manufacturing a bone graft material according to any one of claims 1 to 9, comprising the steps of:
i) providing a mixture comprising a biopolymer matrix component and a biopolymer filler component, preferably in powder form, and a bioactive mineral filler component, preferably in powder and/or particulate form;
ii) mixing the said components with water to obtain a gel-like or paste-like material; and, optionally,
iii) hardening the mixture obtained after step ii) to obtain a hardened bone graft material.

11. The method according to claim 10, **characterized in that** the hardening of step iii) is effected by exposure of the mixture obtained after step ii) to curing/hardening conditions, wherein the nature of said curing/hardening conditions is chosen dependent upon a respective crosslinking agent and optional crosslinking catalyst added to the mixture of step i) or step ii).

12. The method according to claim 10 or claim 11, **characterized in that**
- the biopolymer matrix component comprises or consists of carboxymethyl cellulose (CMC); and/or
- the biopolymer filler component comprises or consists of poly lactic acid (PLA); and/or
- the bioactive mineral filler component comprises or consists of calcium sulphate (CaSO₄) and/or beta tricalcium phosphate (βTCP), preferably both; and/or
- the biopolymer matrix component, the biopolymer filler component and the bioactive mineral filler component are, independently of each other, present in the mixture obtained in step i) in an amount of about 1 to about 65 vol.-%, preferably in an amount of about 5 to about 35 vol.-%, more preferably in an amount of about 10 to about 33 vol.-%, based on the total volume of said mixture; and/or
- the water is distilled or deionized water or a mixture thereof; and/or
- the water has a temperature of about 20 to about 90 °C, preferably about 25 to about 85 °C, more preferably about 30 to about 80 °C, more preferably about 35 to about 75 °C, even more preferably about 40 to about 70 °C, still more preferably about 45 to about 70 °C, most preferably about 50 to about 70 °C, such as about 55 °C to about 70 °C, for instance about 65 °C; and/or
- the mixture obtained in step ii) is exposed to elevated temperatures in the range of about 20 to about 90 °C, preferably about 25 to about 85 °C, more preferably about 30 to about 80 °C, more preferably about 35 to about 75 °C, even more preferably about 40 to about 70 °C, still more preferably about 45 to about 70 °C, most preferably about 50 to about 70 °C, such as about 55 °C to about 70 °C, for instance about 65 °C, preferably for about 15 s to about 5 h, more preferably for about 30 s to about 2 h, more preferably for about 60 s to about 1 h, more preferably for about 1.5 min to about 30 min; and/or
- the method further comprises the addition of at least one additional component selected from the group consisting of osteogenic, osteoconductive and/or osteoinductive materials; anti-inflammatory agents; antibiotics; anti-viral agents; anti-cancer agents; immunosuppressants; enzymes inhibitors; hormones; and proteins, such as bone morphogenic proteins to the mixture of step i) and/or the mixture of step ii).

13. A bone graft material according to any one of claims 1 to 9 or a bone graft material obtainable in a method according to any one of claims 10 to 12 for use in bone grafting related to dental implant placement or dental implant surgery, wherein, preferably, the dental implant surgery comprises craniofacial and/or maxillofacial bone surgery, and the dental implant surgery comprises sinus grafting, ridge augmentation and/or preservation.

14. Kit-of-parts, comprising a biopolymer matrix component, a biopolymer filler component, and a bioactive mineral filler component, **characterized in that** the biopolymer matrix component and the biopolymer filler component are provided in powder form and the bioactive mineral filler component is provided in particulate and/or powder form, the kit optionally further comprising water, more preferably distilled or deionized water or a mixture thereof.

15. The kit-of-parts according to claim 14, **characterized in that** it further comprises at least one crosslinking agent and optionally at least one crosslinking catalyst, preferably selected from hyaluronic acid and carbodiimide, or citric acid.
